Europäisches Patentamt

European Patent Office

Office européen des brevets

(19)

(11) Numéro de publication : **0 626 590 A1**

## (12) DEMANDE DE BREVET EUROPEEN

(21) Numéro de dépôt : **94401098.2**

(22) Date de dépôt : **18.05.94**

(51) Int. Cl.$^5$ : **G01T 1/164**

(30) Priorité : **18.05.93 FR 9305992**

(43) Date de publication de la demande :
**30.11.94 Bulletin 94/48**

(84) Etats contractants désignés :
**BE DE**

(71) Demandeur : **SOPHA MEDICAL**
**41, rue Fourny**
**F-78534 BUC Cedex (FR)**

(71) Demandeur : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **Buvat, Irène**
**Cabinet Ballot-Schmit,**
**7 rue Le Sueur**
**F-75116 Paris (FR)**
Inventeur : **Benali, Habib**
**Cabinet Ballot-Schmit,**
**7 rue Le Sueur**
**F-75116 Paris (FR)**
Inventeur : **Bazin, Jean-Pierre**
**Cabinet Ballot-Schmit,**
**7 rue Le Sueur**
**F-75116 Paris (FR)**
Inventeur : **Di Paola, Robert**
**Cabinet Ballot-Schmit,**
**7 rue Le Sueur**
**F-75116 Paris (FR)**

(74) Mandataire : **Lemoyne, Didier et al**
**Cabinet Ballot-Schmit**
**7, rue Le Sueur**
**F-75116 Paris (FR)**

(54) **Procédé de décomposition d'images scintigraphiques en composantes d'absorption totale et diffusées.**

(57) Selon l'invention, ledit procédé de décomposition comporte les étapes consistant à :
a) échantillonner l'énergie en P échantillons j,
b) enregistrer les valeurs $t_i(j)$ de chaque spectre $\{t_i\}$,
c) construire le tableau Y des $t_i(j)$,
d) déterminer la base de vecteurs propres $\{v_q\}$ ($q = 0, 1, 2..,P-1$) orthogonaux de la matrice Y, classés par ordre de valeurs propres décroissantes,
e) déterminer K spectres fondamentaux $\{f_k\}$ dans l'espace engendré par les Q ($Q \leqq P$) premiers vecteurs propres $\{v_q\}$ en appliquant des contraintes appropriées aux coefficients bkq définis par :

$$\{f_k\} = \sum_{q=0}^{Q} b_{kq} \{v_q\} \quad , \quad b_{k0} = b_0 \; \forall \; k$$

f) projeter les spectres $\{t_i\}$ d'éléments d'image sur la base des spectres fondamentaux $\{f_k\}$ pour obtenir la décomposition recherchée :

$$t_i(j) = \sum_{k=1}^{K} n_k(i) \; f_k(j)$$

Application au traitement des images scintigraphiques.

EP 0 626 590 A1

La présente invention concerne un procédé de décomposition en une composante dite d'absorption totale et une composante dite diffusée, de spectres $\{t_i\}$ d'énergie fournis, pour N éléments i d'image (i = 1, 2,..., N) d'une image scintigraphique, par une caméra à scintillations fonctionnant en mode séquentiel.

L'invention trouve une application particulièrement avantageuse dans le domaine général de la médecine nucléaire, c'est-à-dire l'ensemble des méthodes diagnostiques et thérapeutiques qui nécessitent l'utilisation d'isotopes radioactifs, et notamment la scintigraphie, technique exploratoire visant à représenter au moyen d'une image bidimensionnelle la distribution de la radioactivité dans un organe après administration d'une substance radioactive émettrice gamma, appelée radiotraceur.

Les dispositifs actuellement utilisés pour réaliser une image scintigraphique d'une source radioactive in vivo comportent essentiellement une caméra à scintillations et un système de traitement des signaux fournis par ladite caméra à scintillations.

Le rôle de la caméra à scintillations proprement dite est de détecter les photons gamma issus d'un point appartenant à la zone radioactive. Elle est formée de plusieurs parties :
- un collimateur que doit traverser le rayonnement émis dans l'organisme du patient. Ainsi, seuls arrivent sur le cristal scintillant adjacent les photons dont la direction est pratiquement perpendiculaire à la surface du cristal ; le point d'absorption du photon gamma dans le cristal correspond donc à peu près à son point d'émission, à l'angle d'acceptance du collimateur près. Les collimateurs à trous parallèles sont les plus utilisés.
- le cristal scintillant, généralement de l'iodure de sodium dopé au thallium (Nal (Tl)), matériau capable de convertir l'énergie perdue par une radiation ionisante en une impulsion lumineuse d'intensité proportionnelle à la quantité d'énergie dissipée dans le cristal. Le diamètre du cristal utilisé dans une caméra à scintillations est, par exemple, de 45 cm pour une épaisseur de 1,25 cm.
- un guide de lumière destiné à diriger la lumière des scintillations vers un réseau de tubes photomultiplicateurs.
- des tubes photomultiplicateurs au nombre de 15, 37, 61, 91,..., couvrant la surface de détection. Chaque photomultiplicateur fournit un signal électrique en réponse à l'impulsion lumineuse reçue et ce signal est d'autant plus faible que le tube est éloigné de la scintillation.

Les signaux électriques émis par l'ensemble des tubes photomultiplicateurs sont traités par un calculateur électronique de positionnement de scintillation constitué, de manière connue, par un réseau de pondération sous la forme d'une matrice de résistances permettant de calculer les coordonnées X, Y du photon détecté, indépendamment de son énergie E, proportionnelle à l'amplitude du signal somme. Le principe de positionnement repose sur le fait que chaque tube photomultiplicateur reçoit une quantité de lumière proportionnelle à l'angle solide sous lequel il "voit" la scintillation.

Un processeur reçoit les informations issues de l'appareil de scintigraphie à savoir, essentiellement, les coordonnées X, Y et l'énergie E de chaque événement détecté. Le procédé de décomposition proposé utilise un mode d'acquisition séquentiel dans lequel les coordonnées des positions X, Y et l'énergie E de chaque photon sont transférées directement à la mémoire du processeur sous la forme de liste : X1, Y1, E1, X2, Y2, E2, ...., puis stockées dans la mémoire de masse, un disque par exemple. Aucune image n'est formée durant l'acquisition. Les images sont constituées en différé, après la fin de l'acquisition. Ce mode de fonctionnement est très utile pour certains types de traitement d'images puisqu'il conserve l'ensemble des informations pour chaque événement. Pour une même acquisition, il permet par exemple d'effectuer plusieurs traitements d'images différents à partir des informations originales, simplement par relecture de la liste des caractéristiques des photons détectés.

Outre l'acquisition, le processeur remplit aussi d'autres fonctions :
- correction des imperfections physiques du détecteur, comme l'inhomogénéité de réponse dans le champ de la caméra,
- amélioration de la qualité des images scintigraphiques par des programmes intégrés de soustraction de bruit de fond, de lissage, d'augmentation de contraste, ...,
- traitement quantitatif des images (évaluation de l'activité dans un organe, ...),
- stockage des images et leur transfert sur des unités d'archivage périphériques (bandes magnétiques, ...).

Pour une caméra à scintillations, l'idéal serait de ne détecter que les photons qui, après avoir été émis par le traceur fixé dans l'organe, traversent le patient sans interagir, puis franchissent les trous du collimateur avant de pénétrer dans le cristal scintillant et d'y déposer toute leur énergie. La distribution spectrale de ces photons, dits photons d'absorption totale, serait alors une gaussienne, centrée sur l'énergie d'émission du radioélément, de largeur à mi-hauteur égale à la résolution en énergie du détecteur, typiquement voisine de 10 % pour le $^{99m}$ Tc.

En réalité, les histoires des photons détectés sont très variées. Ils peuvent avoir subi une ou plusieurs diffusions Compton dans l'organisme du patient, ou dans le collimateur. De plus, certains photons interagissent par effet photoélectrique sur le matériau constitutif du collimateur, ce qui entraîne l'émission de photons X caractéristiques de ce matériau. Ceux-ci peuvent ensuite pénétrer dans le cristal et y être totalement absorbés.

Le spectre observé a donc trois composantes :
- une composante due aux photons d'absorption totale non diffusés à l'extérieur du cristal, bien collimatés et entièrement absorbés par le cristal : c'est le pic d'absorption totale centré sur l'énergie d'émission du radioélément,
- une composante de photons diffusés proprement dits, correspondant à la distribution spectrale des photons ayant subi une ou plusieurs diffusions extérieures au cristal. En théorie, leur énergie est par conséquent inférieure à l'énergie d'émission,
- une composante dite "photon X", caractéristique du matériau constitutif du collimateur.

Du fait de la résolution énergétique limitée des caméras à scintillations, ces différentes composantes spectrales se recouvrent partiellement.

La détection de photons diffusés a plusieurs conséquences néfastes :
- elle détériore la résolution spatiale du détecteur, puisqu'elle entraîne une erreur de localisation du point d'émission,
- elle altère la sensibilité du système d'imagerie, c'est-à-dire le rapport du nombre d'événements détectés au nombre d'événements émis,
- elle dégrade le contraste des images,
- elle rend délicate l'interprétation quantitative des images.

Une technique actuellement utilisée pour limiter l'effet des photons diffusés est de former les images à partir des photons contenus dans une fenêtre spectrométrique centrée sur le pic d'absorption totale. Toutefois, cette technique connue ne peut conduire à des résultats satisfaisants puisque le recouvrement des différentes composantes spectrales mentionné ci-dessus introduit une quantité non négligeable de photons diffusés à l'intérieur de la fenêtre spectrométrique.

Aussi, le problème technique à résoudre par l'objet de la présente invention est de proposer un procédé de décomposition conforme au préambule, qui permettrait de séparer les contributions respectives au spectre en énergie des photons d'absorption totale et diffusés, de manière à reconstituer au mieux l'image des seuls photons d'absorption totale et celle des photons diffusés.

La solution au problème technique posé consiste, selon la présente invention, en ce que, dans le but de rechercher pour chaque spectre $\{t_i\}$ une décomposition sur K spectres fondamentaux $\{f_k\}$ (k = 1, 2, ...., K) de la forme :

$$\{t_i\} = \sum_{k=1}^{K} n_k \ (i) \ \{f_k\}$$

où l'un, $\{f_1\}$, desdits spectres fondamentaux, appelé spectre d'absorption totale, est représentatif de ladite composante d'absorption totale, et les K-1, $\{f_k\}$ pour k = 2, ..., K, autres spectres fondamentaux, appelés spectres de photons diffusés, sont représentatifs de ladite composante diffusée, ledit procédé de décomposition comporte les étapes consistant à :

a) échantillonner l'énergie en P échantillons j (j=1, 2, ..., P),
b) enregistrer les valeurs $t_i(j)$ de chaque spectre $\{t_i\}$ d'un élément i d'image pour chaque échantillon j d'énergie,
c) éventuellement, éliminer par seuillage les éléments i d'image dont le spectre $\{t_i\}$ présente un rapport signal sur bruit insuffisant, et regrouper dans M agrégats i' (i'=1, 2, ..., M) des éléments i d'image voisins dont les spectres $\{t_i\}$ sont similaires, de manière à définir, par sommation des spectres $\{t_i\}$ correspondants, M spectres $\{y_{i'}\}$ d'agrégats,
d) construire le tableau Y des $y_{i'}(j)$, valeurs des M spectres $\{y_{i'}\}$ d'agrégats pour les P échantillons j d'énergie, ainsi que la matrice définie par $Y^tDYM$, où D et M sont des matrices diagonales spécifiant une métrique,
e) déterminer la base de vecteurs propres $\{v_q\}$ (q=0, 1, 2, ..., P-1) orthogonaux de la matrice de $Y^tDYM$, classés par ordre de valeurs propres décroissantes, ladite base étant telle que :

$$\{y_{i'}\} = \sum_{q=0}^{P-1} a_{i'q} \{v_q\}$$

f) déterminer les K spectres fondamentaux $\{f_k\}$ dans l'espace engendré par les Q $(Q \leqq P)$ premiers vecteurs propres $\{v_q\}$ en appliquant aux coefficients $b_{kq}$ définis par

$$\{f_k\} = \sum_{q=0}^{Q} b_{kq} \{v_q\} \qquad b_{k0} = b_0 \; \forall \; k$$

avec

$$y'_{i'}(j) = \sum_{q=0}^{Q} a_{i'q} \; v_q \; (j)$$

et

$$y'_{i'}(j) = \sum_{k=1}^{K} c_{i'k} \; f_k \; (j)$$

les contraintes suivantes :
- les $b_{1q}$ (q = 1, 2, ...Q), correspondant au spectre d'absorption totale $\{f_1\}$, sont déterminés par le point de l'espace engendré par les $\{v_q\}$ satisfaisant au mieux la condition de nullité dans certaines plages d'échantillons j d'énergie,
- les $b_{kq}$ (k = 2, ...,k), correspondant aux spectres de photons diffusés, sont déterminés sous les conditions suivantes :
  . condition de positivité des spectres : $f_k$ (j) $\geqq$ 0 $\forall$j, k=2, ..., K
  . condition de positivité des images : $C_{i'k} \geqq 0 \; \forall i',k$
  . condition de dissimilarité entre $\{f_1\}$ et $\{f_k\}$ k $\neq$ 1

g) projeter les spectres $\{t_i\}$ d'éléments d'image sur la base des spectres fondamentaux $\{f_k\}$ pour la détermination des $n_k$ (i) qui minimisent la différence

$$e_{ij} = t_i(j) - \sum_{k=1}^{K} n_k(i) \; f_k \; (j)$$

Comme on le verra en détail plus loin, une des principales caractéristiques du procédé de décomposition de l'invention est qu'il constitue une méthode d'analyse adaptative en ce sens qu'il s'adapte automatiquement aux conditions d'acquisition. En particulier, le procédé conforme à l'invention n'exige aucune hypothèse initiale quant à la géométrie d'acquisition, aux détecteurs, à la corpulence du patient, au centre et à la largeur du pic d'absorption totale.

Le procédé de décomposition, objet de l'invention, apparaît donc comme une méthode universelle pouvant connaître une grande variété d'applications, en particulier :
- les examens scintigraphiques planaires, statiques ou dynamiques,
- les examens scintigraphiques tomographiques statiques : tomographie d'émission à simple photon (Single Photon Emission Computed Tomography : SPECT). La séquence d'images est obtenue en faisant tourner la tête de détection de la caméra sur une orbite autour du patient. Contrairement à la scintigraphie plane, la tomographie permet une investigation tridimensionnelle de l'organe. L'exploration du volume reconstruit se fait le plus souvent au moyen de coupes à travers le volume,

- les examens mono ou multiisotopiques.

La description qui va suivre en regard des dessins annexés, donnés à titre d'exemples non limitatifs, fera bien comprendre en quoi consiste l'invention et comment elle peut être réalisée.

La figure 1 est un schéma de principe d'une caméra à scintillations.

La figure 2 est un diagramme montrant le spectre en énergie du signal scintigraphique, sur lequel apparaissent les contributions respectives des photons d'absorption totale et des photons diffusés.

La figure 3 est un schéma montrant le principe de décomposition du procédé, objet de l'invention.

La figure 4 est un schéma représentant le mode d'acquisition des données d'une caméra à scintillations fonctionnant conformément au procédé de décomposition de l'invention.

La figure 5 est un schéma illustrant l'opération de seuillage effectuée lors de la mise en oeuvre du procédé conforme à l'invention.

La figure 6 est un schéma illustrant l'opération d'agrégation effectuée lors de la mise en oeuvre du procédé conforme à l'invention.

La figure 7 montre la formation du tableau Y des $y_{l'}$ (j), valeurs des M spectres d'agrégats pour P échantillons d'énergie.

La figure 8 est un diagramme construit sur les vecteurs propres $v_1$ et $v_2$ issus de l'analyse orthogonale du tableau Y de la figure 7, et dans lequel sont portés les points représentatifs du spectre $\{y_{l'}\}$ de chaque agrégat d'éléments d'image.

La figure 9 est un diagramme construit sur la même base de vecteurs propres que la figure 8, dans lequel sont portés les points représentatifs des spectres fondamentaux recherchés résultant de l'analyse oblique effectuée à la suite de l'analyse orthogonale illustrée la figure 8.

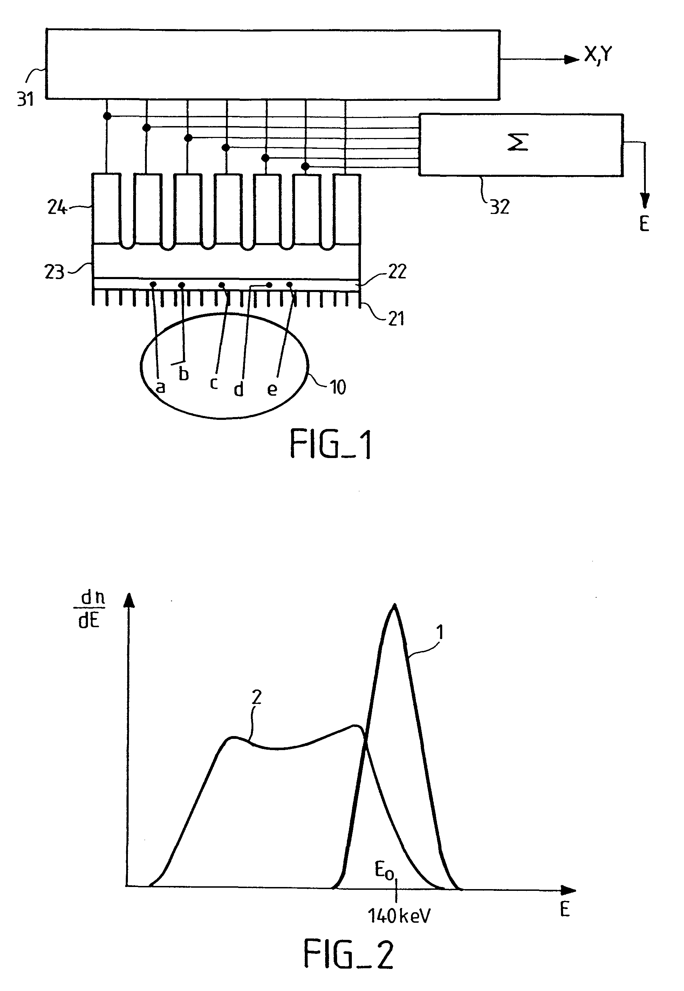

La figure 1 donne de façon schématique le principe de fonctionnement d'une caméra à scintillations. Le rayonnement gamma émis par un traceur radioactif administré au patient 10 traverse un collimateur 21 à canaux parallèles avant d'atteindre un cristal scintillant 22 en NaI (TI) par exemple. Ce cristal convertit l'énergie perdue par le rayonnement gamma incident en une impulsion lumineuse transmise à une matrice de tubes photomultiplicateurs 24 par un guide 23 de lumière.

Les signaux électriques issus des différents tubes photomultiplicateurs sont, d'une part, traités de manière connue par un calculateur électronique 31 de positionnement comprenant une matrice de résistances fournissant les coordonnées X, Y de la scintillation détectée, et, d'autre part, sommées par un circuit 32 de sommation de façon à fournir un signal de sortie proportionnel à l'énergie E du photon détecté. La figure 2 montre un exemple typique de spectre en énergie enregistré par une caméra à scintillations conforme à celle représentée sur la figure 1, donnant la densité spectrale d'événements dn/dE en fonction de l'énergie E.

Comme l'indique la figure 2, le spectre en énergie du signal scintigraphique résulte de la superposition du spectre 1 des photons d'absorption totale, ayant déposé toute leur énergie dans le cristal, correspondant aux trajectoires a et d des photons gamma montrées à la figure 1, et du spectre 2 des photons diffusés, correspondant aux trajectoires b,c et e de la figure 1. En b les photons gamma subissent une diffusion Compton dans l'organisme même du patient 10 ; ceux qui suivent la trajectoire c sont diffusés par le collimateur 21, tandis qu'en e la diffusion a pour origine l'interaction par effet photoélectrique des photons gamma avec le matériau constituant le collimateur 21.

On peut voir sur la figure 2 la décomposition théorique du spectre enregistré en spectre 1 des photons d'absorption totale et spectre 2 des photons diffusés. Le spectre 1 des photons d'absorption totale est représentée par une courbe gaussienne centrée sur l'énergie $E_o$ d'émission du radioélément utilisé, ici 140 keV pour le technetium 99 m et dont la largeur à mi-hauteur est déterminée par la résolution en énergie de la caméra. Le spectre 2 des photons diffusés a une forme qui dépend des contributions respectives des différentes causes de diffusion énumérées ci-dessus ; il est théoriquement situé à des énergies plus basses que le spectre primaire avec toutefois un recouvrement relativement important, dû à la résolution en énergie limitée du détecteur.

Il a été rappelé plus haut combien la présence de photons diffusés pouvait être préjudiciable à la qualité de l'image scintigraphique fournie par la caméra. Aussi, le procédé qui va maintenant être décrit en détail a pour but de séparer dans le spectre d'énergie de la figure 2 la contribution des photons d'absorption totale, dite composante d'absorption totale, de celle des photons diffusés, dite composante diffusée.

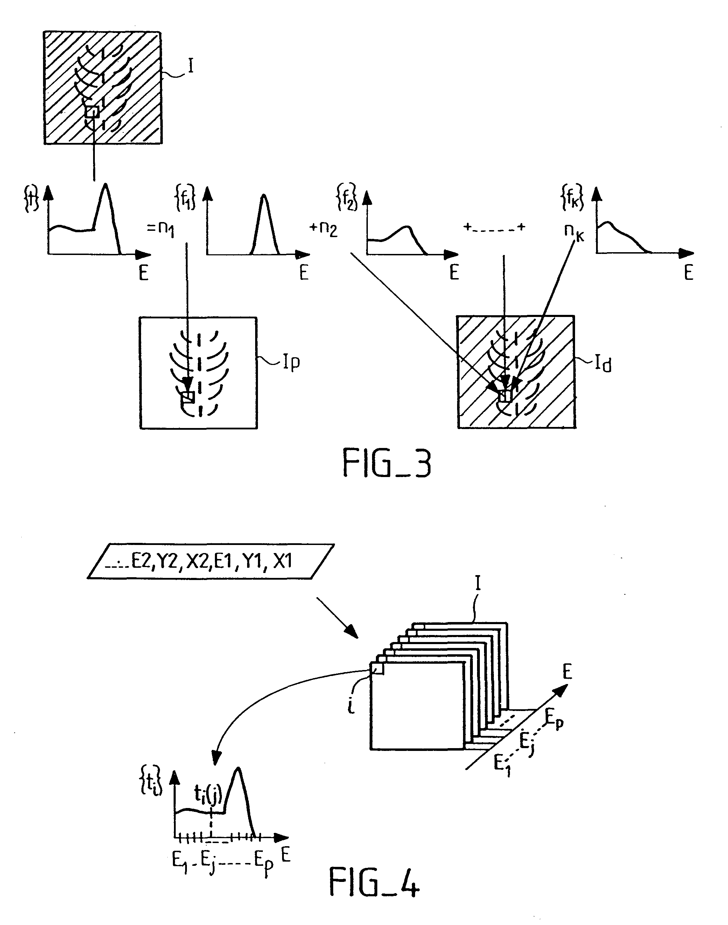

Selon le principe illustré schématiquement sur la figure 3, le spectre associé à chaque élément d'image, généralement noté $\{t_j\}$, acquis par la caméra à scintillations est décomposé en K spectres fondamentaux, à savoir un spectre d'absorption totale $\{f1\}$, représentatif de ladite composante d'absorption totale, et K-1 spectres de photons diffusés {f2}, ..., {fk}, représentatif de ladite composante diffusée, chacun de ces spectres étant affecté d'un coefficient de pondération $n_k$ (i).

Sur la figure 3, on a représenté, d'une part, une image scintigraphique I qui serait obtenue sans l'intervention du procédé, objet de l'invention, et, d'autre part, la décomposition de cette image en deux images $I_p$ et $I_d$, l'une, $I_p$, de qualité image améliorée étant reconstituée à partir des seuls photons d'absorption totale {t}, tandis que l'autre, $I_d$, serait l'image, profondément dégradée, qui serait obtenue à partir des photons diffusés.

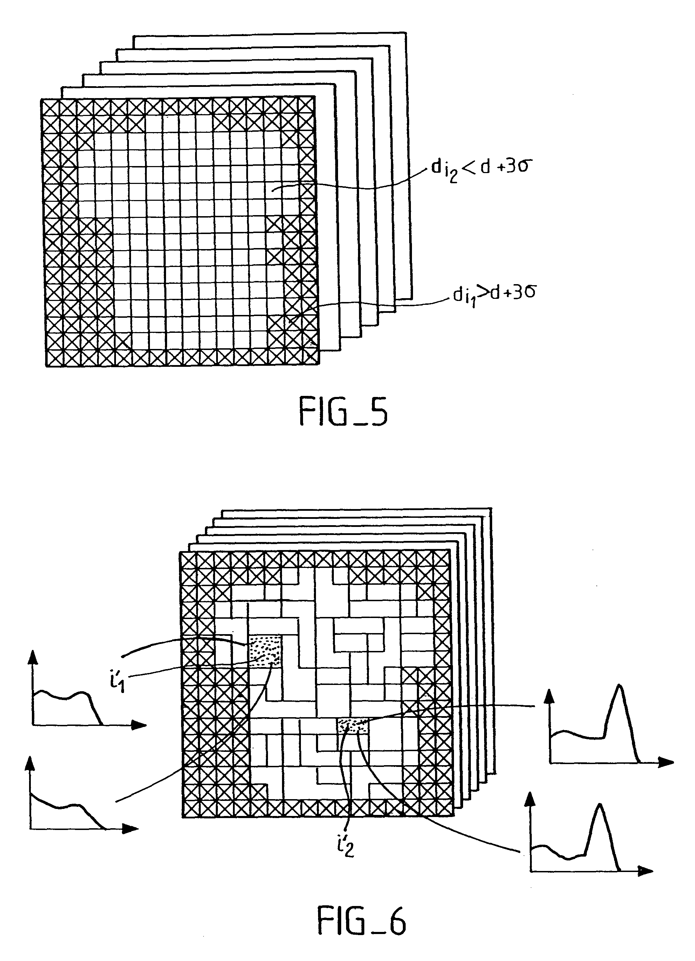

On a montré sur la figure 4 de quelle manière l'acquisition des spectres était effectuée.

Tout d'abord l'image scintigraphique I est divisée en N éléments i d'image (i variant de 1 à N), ce qui se traduit par une quantification des coordonnées X et Y de position des événements détectés. D'autre part, l'énergie est échantillonnée en P échantillons j (j variant de 1 à P).

La caméra à scintillations concernée par le procédé de l'invention fonctionne en mode séquentiel, c'est-à-dire que les données, à savoir les coordonnées de position X, Y et l'énergie E de chaque photon détecté, sont acquises sous forme de liste : X1, Y1, E1, X2, Y2, E2..,X1, Y1 et E1 représentant les coordonnées et l'énergie du premier événement détecté. On peut ainsi, pour chaque élément i d'image, construire le spectre d'énergie {$t_i$} en portant en ordonnées le nombre de photons $t_i(j)$ détectés pour chaque échantillon Ej d'énergie. Les valeurs ti(j) ainsi déterminées sont conservées dans la mémoire du processeur de la caméra.

Le procédé de décomposition de l'invention a donc pour objet la recherche, pour chaque spectre {$t_i$} d'élément d'image, d'une décomposition sur K spectres fondamentaux {$f_k$} de la forme :

$$\{t_i\} = \sum_{k=1}^{K} n_k (i) \{f_k\}$$

expression dans laquelle à la fois les {$f_k$} et les $n_k$ (i) sont à déterminer.

Il convient de noter que le nombre K des spectres fondamentaux est imposé par l'expérimentateur en fonction des conditions physiques d'acquisition des données. Dans le cas du technetium 99 m on pourra choisir K = 3 soit 1 spectre d'absorption totale et 2 spectres de photons diffusés.

Une étape préliminaire du procédé, objet de l'invention, consiste à préparer les données de manière à disposer de spectres présentant un meilleur rapport signal/bruit. Cette étape préparatoire peut être effectuée à l'aide de deux opérations successives : une opération dite de seuillage et une opération dite d'agrégation.

Le seuillage, schématisé sur la figure 5, a pour but d'éliminer les éléments i d'image dont le spectre {$t_i$} présente trop de bruit. A titre d'exemple, l'opération de seuillage peut être réalisée de la manière suivante :
- on construit le tableau T des $t_i$ (j) :

$$T = \begin{bmatrix} t_1(1) & \cdots & t_1(j) & \cdots & t_1(P) \\ \cdot & & & & \\ \cdot & & & & \\ \cdot & & & & \\ t_i(1) & \cdots & t_i(j) & \cdots & t_i(P) \\ \cdot & & & & \\ \cdot & & & & \\ \cdot & & & & \\ t_N(1) & \cdots & t_N(j) & \cdots & t_N(P) \end{bmatrix}$$

Le tableau T est une matrice NxP dont on observera qu'une ligne i correspond au spectre {$t_i$} d'énergie décomposé sur les P échantillons, alors qu'une colonne j de ce tableau représente l'image partielle formée par l'ensemble des N éléments d'image pour l'échantillon $E_j$ d'énergie.

A partir du tableau T, on construit la matrice définie par

$$T^tD'TM'$$

où $T^t$ désigne la matrice transposée de T. D' et M' sont des matrices diagonales spécifiant une métrique. D' est une matrice définissant les poids associés à chaque spectre, poids fonctions de l'intensité de chaque spectre. De manière similaire, M' est une matrice définissant les poids associés à chaque image, poids fonctions de l'intensité de chaque image. Ces poids permettent de donner une importance plus grande aux spectres, respectivement aux images, d'intensité plus élevée. D' et M' correspondent donc à la prise en compte d'une métrique associée aux données.

Les éléments de la matrice diagonale D' sont fonctions des intensités des spectres, à savoir :

$$t_i = \sum_{j=1}^{P} t_i(j)$$

Symétriquement, les éléments de la matrice diagonale M' sont fonctions des intensités des images :

$$t_j = \sum_{i=1}^{N} t_i(j)$$

- on détermine la base des P vecteurs propres {wq} (q = 0, 1, ...P-1) orthogonaux de la matrice $T^tD'TM'$, classés par ordre de valeurs propres décroissantes. La base des vecteurs {wq} est donc reliée linéairement à la base initiale des {tᵢ} par la relation :

$$t_i(j) = \sum_{q=0}^{P-1} \alpha_{iq} \, w_q(j)$$

dans laquelle les $\alpha_{iq}$ sont des coefficients de changement de base dont les valeurs sont connues.
- on calcule ensuite pour chaque élément i d'image un paramètre $d_i$ de la forme

$$d_i = \sum_{q=Q+1}^{P-1} \alpha^2{}_{iq} \, \frac{t}{t_i}$$

où

$$t = \sum_{ij} \sum t_i(j)$$

est le nombre total de photons,
et

$$t_i = \sum_j t_i(j)$$

est l'intensité du spectre $t_i$,

Q+1 étant l'ordre à partir duquel les $w_q$ représentent essentiellement du bruit, l'information utile étant concentrée dans les Q premiers vecteurs propres $w_q$. De façon pratique, on pourra prendre Q = K-1.

Le paramètre $d_i$ qui est représentatif de la contribution du spectre $\{t_i\}$ au bruit, est d'autant plus grand que cette contribution est grande. Par conséquent, seront éliminés les éléments $i_1$ d'image dont le paramètre $di_1$ sera supérieur à une valeur limite donnée. A l'inverse, les éléments $i_2$ d'image dont le paramètre $di_2$ est inférieur à cette valeur limite seront conservés pour le traitement ultérieur.

- pour établir la valeur limite de seuillage, on calcule la moyenne d des paramètres $d_i$ et leur écart-type $\sigma$,
- puis on élimine les éléments $i_1$ d'image pour lesquels

$$di_1 > d + 3\sigma$$

et on conserve les éléments $i_2$ d'image pour lesquels,

$$di_2 < d + 3\sigma$$

On calcule de nouveau d et $\sigma$ à partir des éléments i restants, et on répète le processus tant que l'on élimine des éléments i.

Le résultat d'un tel seuillage est montré sur la figure 5.

Après seuillage, on peut également effectuer une opération d'agrégation des éléments d'image conservés en regroupant, comme l'indique la figure 6, des éléments d'image voisins pour constituer des agrégats généralement notés i', au nombre de M (i'= 1,2,..,M), dont les spectres, $\{y_{i'}\}$ sont chacun la somme des spectres $\{t_i\}$ des éléments i d'image participant à l'agrégat considéré. On peut assortir l'agrégation d'une condition de similarité de forme des spectres $\{t_i\}$ des éléments d'image voisins. Cette condition est illustrée sur la figure 6 où il apparaît que l'agrégat $i'_1$ est constitué à partir d'éléments d'image présentant essentiellement des spectres de photons diffusés, alors que l'agrégat $i'_2$ regroupe des éléments d'image dont les spectres comporte une importante composante d'absorption totale.

Il faut souligner que les opérations de seuillage et d'agrégation dont le seul but est de ne traiter que des éléments d'image significatifs du point de vue du signal, ne sont pas essentielles pour le procédé de décomposition de l'invention. Elles pourraient même être omises dans le cas où les conditions d'acquisition des données sont favorables en ce qui concerne le rapport signal/bruit. De même, on peut également envisager de n'effectuer que l'une ou l'autre de ces opérations, dans la mesure où elles peuvent être menées de façon indépendante.

L'étape suivante du procédé, objet de l'invention, est semblable à celle précédemment décrite qui a permis de définir l'opération de seuillage.

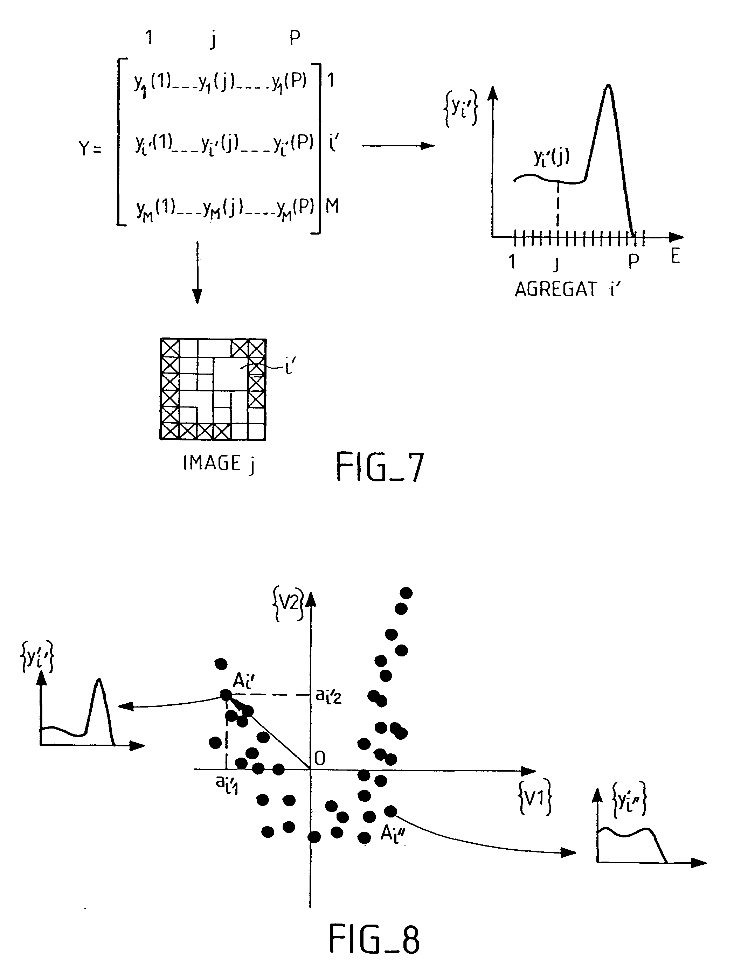

Conformément à la figure 7, on construit le tableau Y des MxP valeurs $y_{i'}(j)$ des M spectres $\{y_{i'}\}$ d'agrégats pour les P échantillons j d'énergie. Chaque ligne i' de la matrice Y représente le spectre $\{y_{i'}\}$ d'énergie de l'agrégat i', tandis que chaque colonne j reconstitue l'image en terme d'agrégats i' pour l'échantillon Ej d'énergie.

A partir du tableau Y, on calcule la matrice de donnée par :

$$Y^t DYM$$

$Y^t$ désignant la matrice transposée de Y, D et M étant des matrices diagonales spécifiant une métrique. De manière analogue aux matrices D' et M' définies plus haut, les éléments des matrices D et M sont des fonctions des intensités respectives des spectres et des images :

$$Y_{i'} = \sum_{j=1}^{P} Y_i (j)$$

$$Y_j = \sum_{i'=1}^{M} Y_{i'} (j)$$

On détermine la base des vecteurs propres orthogonaux $\{v_q\}$ de la matrice $Y^t DYM$, avec q variant de 0 à P-1. Les vecteurs propres sont supposés être classés par ordre de valeurs propres décroissantes. Il existe une relation linéaire entre la base des vecteurs propres $\{v_q\}$ et celle des vecteurs $\{y_{i'}\}$, ce qui se traduit par l'expression :

$$y_{i'}(j) = \sum_{q=0}^{P-1} a_{i'q}\, v_q\,(j)\quad(1)$$

les coefficients $a_{i'q}$ sont les éléments de la matrice de passage d'une base à l'autre.

Comme cela a déjà été fait pour l'opération de seuillage, on peut limiter le développement (1) aux Q premiers vecteurs propres $(v_q)$, les P-Q restant n'étant supposés représenter que du bruit. Dans la suite, on ne prendra en compte que les spectres "approchés" $\{y'_{i'}\}$ définis par

$$y'_{i'}(j) = \sum_{q=0}^{Q} a_{i'q}\, v_q\,(j)$$

Avec K=3 et Q= K-1 = 2, cette dernière relation se traduit par la relation suivante entre les spectres, ou vecteurs de base :

$$\{y_{i'}\} = a_0\,\{v_0\} + a_{i'1}\,\{v_1\} + a_{i'2}\,\{v_2\}$$

La figure 8 donne une représentation graphique de cette analyse, appelée analyse orthogonale du fait de l'orthogonalité des vecteurs propres $\{v_q\}$. Dans le repère constitué par les deux vecteurs $\{v_1\}$ et $\{v_2\}$, chaque spectre d'agrégat tronqué y $\{y'_{i'}\}$ est représenté par un vecteur $OA_i'$ dont les composantes sont les coefficients $a_{i'1}$ et $a_{i'2}$. Chaque spectre d'agrégat y ayant la même coordonnée $a_0$ sur le vecteur $\{v_0\}$, celui-ci n'est pas représenté. En effet, dans la décomposition des spectres

$$\{y_{i'}\} = \sum_{q=0}^{P-1} a_{i'q}\,\{v_q\},$$

$\{v_0\}$ représente le spectre moyen, pour la métrique définie par M et D. Chaque spectre $\{y_i\}$ s'écrit donc comme la somme du spectre moyen ($a_{i'0}=1$ quel que soit i') et d'une combinaison linéaire des vecteurs propres $\{v_q\}$ pondérés par des coefficients $a_{i'q}$. Les vecteurs propres $\{v_q\}$ ($q{\geq}1$) décrivent la variance des spectres autour du spectre moyen, c'est-à-dire l'écart des spectres au spectre moyen.

Il est important de préciser à ce stade que la décomposition résultant de l'analyse orthogonale qui vient d'être faite ne répond pas à la décomposition initialement recherchée en terme de spectres des photons d'absorption totale et de photons diffusés. En effet, les spectres de base $\{v_q\}$ n'ont aucune signification physique qui pourrait les rattacher à tel ou tel phénomène, ce ne sont que des entités mathématiques résultant d'une opération classique de diagonalisation de matrice.

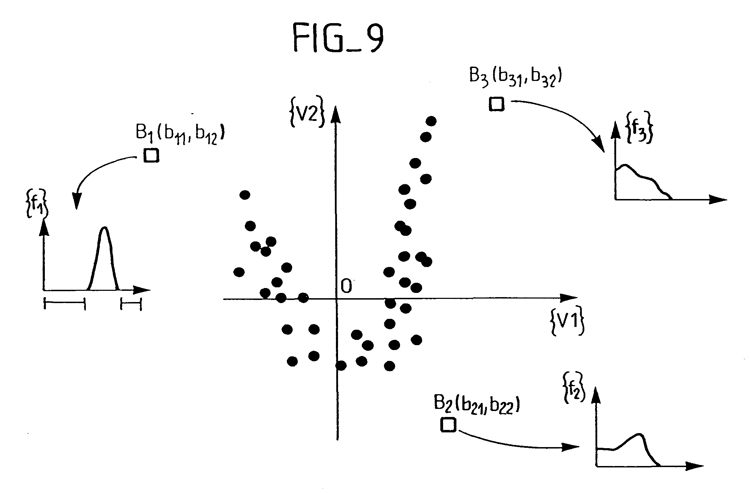

Par contre, on suppose que les spectres fondamentaux $\{f_k\}$ recherchés, physiquement significatifs, se décomposent sur la base des $\{v_q\}$ et donc sont contenus dans l'espace, ici le plan engendré par ces vecteurs. C'est ce que montre la figure 9 dans le cas où le nombre K de spectres fondamentaux est égal à 3. Chacun de ces spectres est représentée par un point $B_k$ (k = 1,2,3) dans le plan $(v_1,v_2)$ dont les coordonnées sont des nombres $b_{k1}$, $b_{k2}$ qui interviennent comme coefficients dans le développement général :

$$\{f_k\} = \sum_{q=0}^{Q} b_{kq}\,\{v_q\}$$

$b_{k0} = b_0$, quel que soit k, le vecteur $v_0$ n'est pas représenté.

Compte tenu de la relation (1) précédente, les spectres tronqués d'agrégats $\{y'_{i'}\}$ sont définis sur la base des spectres fondamentaux par :

$$y'_{i'}(j) = \sum_{k=1}^{K} c_{i'k} \, f_k(j)$$

Les coefficients $c_{i'k}$ ne sont pas indépendants, ils s'expriment en fonction des $a_{i'q}$ et des $b_{kq}$.

Le problème qui reste à résoudre, appelé analyse oblique, est de trouver la position, jusque là inconnue, des points $B_1$, $B_2$, et $B_3$ (plus généralement les $B_k$) dans le plan $\{v_1, v_2\}$ c'est-à-dire déterminer les coefficients $b_{kq}$. Pour résoudre ce problème, on impose des contraintes physiques aux spectres recherchés. Ces contraintes sont les suivantes :

- les coefficients $b_{1q}$ (q = 1,2,...,Q), correspondant au spectre d'absorption totale $\{f_1\}$, sont déterminés par le point $B_1$ de l'espace des $\{v_q\}$ satisfaisant au mieux la condition de nullité dans certaines plages d'échantillons j d'énergie. Dans le cas d'un spectre d'absorption totale à un seul pic gaussien, la condition de nullité porte sur les domaines spectraux situés de part et d'autre du pic (figure 9). Si le radioélément utilisé présentait deux pics d'émission, une troisième condition de nullité entre les pics viendrait s'ajouter aux deux premières.
- les coefficients $b_{kq}$ (k = 2, ..., K), correspondant aux spectres "diffusés", sont déterminés sous les conditions suivantes :
  - . condition de positivité des spectres : $f_k(j) \geqq 0 \; \forall j$
  - . condition de positivité des images : $C_{i'k} \geqq 0 \; \forall i',k$
  - . condition de dissimilarité entre $\{f_1\}$ et les $\{f_k\}$ $k \neq 1$

On obtient ainsi la valeur des coefficients $b_{kq}$, donc les spectres fondamentaux $\{f_k\}$.

Les spectres $\{f_k\}$ étant déterminés, il suffit maintenant de réaliser la décomposition recherchée en projetant en spectres $\{t_i\}$ d'éléments d'image, et non plus ici d'agrégats, sur la base des $\{f_k\}$ pour déterminer les coefficients $n_k(i)$ de la décomposition.

Cette recherche peut être effectuée en déterminant les $n_k(i)$ qui minimisent, au sens des moindres carrés, la différence $e_{ij}$ définie par

$$e_{ij} = t_i(j) - \sum_{k=1}^{K} n_k(i) f_k(j)$$

En conclusion, on a décrit un procédé qui consiste à établir une base de spectres fondamentaux adaptée à chaque ensemble de données correspondant à une acquisition, en ce sens qu'à un ensemble de données est associée une base spécifique de spectres fondamentaux.

L'intérêt de disposer d'une base adaptée est primordial puisque le phénomène de diffusion (angles préférentiels de diffusion, proportion de photons diffusés, ...) dépend de la géométrie du milieu diffusant (forme, taille, corpulence du patient) et de sa composition (densité du milieu, muscle, os,...). Le phénomène de diffusion dépend donc de la morphologie du patient, de l'incidence de l'examen (face antérieure, postérieure, scintigraphie thoracique, abdominale,...), de la distance patient-détecteur, etc.

## Revendications

**1 -** Procédé de décomposition en une composante dite d'absorption totale et une composante dite diffusée, de spectres $\{t_i\}$ d'énergie fournis, pour N éléments i d'image (i=1, 2, ... N) d'une image scintigraphique, par une caméra à scintillations fonctionnant en mode séquentiel, caractérisé en ce que, dans le but de rechercher pour chaque spectre $\{t_i\}$ une décomposition sur K spectres fondamentaux $\{f_k\}$ (k = 1, 2, ..., K) de la forme :

$$\{t_i\} = \sum_{K=1}^{K} n_k(i) \, \{f_k\}$$

où l'un, $\{f_1\}$, desdits spectres fondamentaux, appelé spectre d'absorption totale, est représentatif de ladite composante d'absorption totale, et les K-1, $\{f_k\}$ pour K=2, ..., K, autres spectres fondamentaux, appelés spectres de photons diffusés, sont représentatifs de ladite composante diffusée, ledit procédé de décomposition comporte les étapes consistant à :

a) échantillonner l'énergie en P échantillons j (j=1, 2, ..., P),

b) enregistrer les valeurs $t_i(j)$ de chaque spectre $\{t_i\}$ d'un élément i d'image pour chaque échantillon j d'énergie,

c) éventuellement, éliminer par seuillage les éléments i d'image dont le spectre $\{t_i\}$ présente un rapport signal sur bruit insuffisant, et regrouper dans M agrégats i' (i' = 1, 2, ...M) des éléments i d'image voisins dont les spectres $\{t_i\}$ sont similaires, de manière à définir, par sommation des spectres $\{t_i\}$ correspondants, M spectres $\{y_{i'}\}$ d'agrégats,

d) construire le tableau Y des $y_{i'}(j)$, valeurs des M spectres $\{y_{i'}\}$ d'agrégats pour les P échantillons j d'énergie, ainsi que la matrice définie par $Y^tDYM$, D et M étant des matrices diagonales spécifiant une métrique,

e) déterminer la base de vecteurs propres $\{v_q\}$ (q= 0, 1, 2.., P-1) orthogonaux de la matrice $Y^tDYM$, classés par ordre de valeurs décroissantes, ladite base étant telle que :

$$\{y_{i'}\} = \sum_{q=0}^{P-1} a_{i'q}\{v_q\}$$

f) déterminer les K spectres fondamentaux $\{f_k\}$ dans l'espace engendré par les Q (Q≦P) premiers vecteurs propres $\{v_q\}$ en appliquant aux coefficients $b_{kq}$ définis par :

$$\{f_k\} = \sum_{q=0}^{Q} b_{kq}\{v_q\} \quad , \quad b_{k0} = b_0 \; \forall \, k$$

avec

$$y'_{i'}(j) = \sum_{q=0}^{Q} a_{i'q}\, v_q(j)$$

et

$$y'_{i'}(j) = \sum_{k=1}^{K} c_{i'k}\, f_k(j)$$

les contraintes suivantes :

- les $b_{1q}$ (q=1,2, ...,Q), correspondant au spectre d'absorption totale $\{f_1\}$, sont déterminés par le point de l'espace des $\{v_q\}$ satisfaisant au mieux la condition de nullité dans certaines plages d'échantillons j d'énergie,
- les $b_{kq}$ (k=2, ...K), correspondant aux spectres de diffusés, sont déterminés sous les conditions suivantes :
  . condition de positivité des spectres : $f_k(j) ≧ 0 \; \forall j, k=2, ...K$
  . condition de positivité des images : $c_{i'k} ≧ 0 \; \forall i', k$
  . condition de dissimilarité entre $\{f_1\}$ et $\{f_k\}$ $k \neq 1$

g) projeter les spectres $\{t_i\}$ d'éléments d'image sur la base des spectres fondamentaux $\{f_k\}$ pour la détermination des $n_k(i)$ qui minimisent la différence

$$e_{ij} = t_i(j) - \sum_{k=1}^{K} n_k(i) \, f_k(j)$$

**2 -** Procédé de décomposition selon la revendication 1, caractérisé en ce que ladite étape de seuillage des éléments i d'image comporte les opérations consistant à :
- construire le tableau T des $t_i(j)$, valeurs des N spectres $\{t_i\}$ d'éléments d'image pour les P échantillons j d'énergie, ainsi que la matrice définie par $T^tD'TM'$, D' et M' étant des matrices diagonales spécifiant une métrique,
- déterminer la base de vecteurs propres $\{w_q\}$ (q=1, 2,...P) orthogonaux de la matrice $T^tD'TM'$, classés par ordre de valeurs propres décroissantes, ladite base étant telle que :

$$t_i(j) = \sum_{q=0}^{P-1} \alpha_{iq} \, w_q \, (j)$$

- calculer pour chaque élément i d'image un paramètre $d_i$ de la forme

$$d_i = \sum_{q=Q+1}^{P-1} \alpha^2_{iq} \, \frac{t}{t_i}$$

où

$$t = \sum_{ij} \Sigma t_i(j)$$

est le nombre total de photons,
et

$$t_i = \sum_{j} t_i(j)$$

est l'intensité du spectre $t_i$,
- calculer la valeur moyenne d des paramètres $d_i$ et leur écart-type $\sigma$,
- éliminer les éléments i d'image pour lesquels $d_i > d + 3\sigma$,
- calculer de nouveau d et $\sigma$ pour les valeurs $d_i$ restantes,
- éliminer les éléments i d'image pour lesquels $d_i > d + 3\sigma$,
- répéter la procédure tant qu'on élimine des éléments d'image.

**3 -** Procédé de décomposition selon l'une des revendications 1 ou 2, caractérisé en ce que Q = K-1

**4 -** Procédé de décomposition selon l'une quelconque des revendications 1 à 3, caractérisé en ce que K=3 pour une caméra à scintillations utilisée pour la détection des photons émis par le 99 mTc.

FIG_1

FIG_2

FIG_3

FIG_4

$d_{i_2} < d + 3\sigma$

$d_{i_1} > d + 3\sigma$

FIG_5

$i'_1$

$i'_2$

FIG_6

FIG_7

FIG_8

# FIG_9

Office européen
des brevets

**RAPPORT DE RECHERCHE EUROPEENNE**

Numero de la demande

EP 94 40 1098

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | FR-A-2 663 127 (ELSCINT LTD) * revendications 1-23 * | 1 | G01T1/164 |
| A | US-A-4 878 186 (D.GAGNON) | | |

|  |
|---|
| **DOMAINES TECHNIQUES RECHERCHES (Int.Cl.5)** |
| G01T G06F |

Le présent rapport a été établi pour toutes les revendications

| Lieu de la recherche | Date d'achèvement de la recherche | Examinateur |
|---|---|---|
| LA HAYE | 6 Juillet 1994 | Van den Bulcke, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)